# EUROPÄISCHE PATENTSCHRIFT

(11) **EP 1 976 576 B1**
(45) Veröffentlichungstag und Bekanntmachung des Hinweises auf die Patenterteilung: **18.04.2012**
(21) Anmeldenummer: 07700113.9
(22) Anmeldetag: 12.01.2007
(51) Int. Cl.: A61M 1/00, A61G 7/05

(54) **BEFESTIGUNGSVORRICHTUNG FÜR DRAINAGEBEHÄLTER**
FASTENING DEVICE FOR A DRAINAGE CONTAINER
DISPOSITIF DE FIXATION POUR RÉCIPIENT DE DRAINAGE

(30) Priorität: 27.01.2006 CH 141062006
(43) Veröffentlichungstag der Anmeldung: 08.10.2008
(73) Patentinhaber: Medela Holding AG, 6340 Baar (CH)
(72) Erfinder: CHRISTEN, Lukas, 6280 Hochdorf (CH); LARSSON, Michael, CH-6300 Zug (CH)
(74) Vertreter: Clerc, Natalia
(86) Internationale Anmeldenummer: PCT/CH2007/000015
(87) Internationale Veröffentlichungsnummer: WO 2007/085100

(56) Entgegenhaltungen:
- EP-A1- 0 861 668
- EP-A2- 0 092 313
- WO-A-01/49344
- WO-A-83/01767
- WO-A-93/18803
- WO-A-96/34636
- GB-A- 2 333 459
- JP-A- 2000 079 162
- US-A- 3 915 189
- US-A- 3 955 572
- US-A- 4 101 043
- US-A- 5 356 038
- US-A1- 2002 111 592
- US-B1- 6 494 869
- US-B1- 6 637 707

## Beschreibung

### Technisches Gebiet

Die Erfindung betrifft einen Drainagebehälter gemäss Oberbegriff des Patentanspruchs1.

### Stand der Technik

Drainagebehälter sind Auffangbehälter für Körperflüssigkeiten, welche beim Absaugen im Rahmen medizinischer Behandlungen abfallen. Die Körperflüssigkeit wird mittels einer Vakuumpumpe über eine patientenseitige Drainageleitung in den Behälter geleitet. Der Behälter weist hierfür einen Anschluss für die patientenseitige Drainageleitung und einen Anschluss für die Saug- oder Vakuumpumpe oder für das Zentralvakuum auf

Es sind Drainagebehälter bekannt, welche einen starren Aussenbehälter, einen diesen verschliessenden Deckel mit den oben genannten Anschlüssen und einen am Deckel anbringbaren bzw. angebrachten Drainage- oder Auffangbeutel aufweisen. Der Aussenbehälter ist dabei wiederverwendbar, der Drainagebeutel wird einmalig verwendet und dann fachgerecht entsorgt bzw. vorgängig gewaschen und dann erst entsorgt. Ein derartiger Drainagebehälter ist beispielsweise aus EP 0'861'668 bekannt. Weitere Drainagebehälter sind in US 3'680'560, WO 94/14045, JP 2000 079162 und US 5'470'324 beschrieben.

Diese Drainagebehälter werden üblicherweise an eine Schiene gehängt, welche entweder am Krankenbett selber angebracht ist, an einem fahrbaren Gestell angeordnet ist oder an einer benachbarten Wand dazu verläuft. Als Befestigungsvorrichtungen für Drainagebehälter lassen sich beispielsweise Befestigungsklammern verwenden, welche die Schiene einklemmen. Da die Schienen üblicherweise ein Normgrösse aufweisen, lässt sich dieselbe Grösse von Befestigungsklammern in unterschiedlichen Spitälern verwenden. Der Drainagebehälter wird dabei in die Befestigungsklammer eingehängt. Die Befestigungsklammer gewährleistet somit während der Drainage eine sichere Halterung des Behälters.

Um einen vollen Drainagebeutel durch einen leeren zu ersetzen, werden beide Hände benötigt, da einerseits der Behälter festgehalten und andererseits die Deckelverschlüsse gelöst und der Beutel mit dem Deckel herausgehoben werden muss.

US 3 915 189 beschreibt einen starren Drainagebehälter mit Deckel, bei welchem die Drainageflüssigkeit direkt und ohne Innenbeutel im Behälter gesammelt wird. Auch in US 3 955 752 wird die Drainageflüssigkeit direkt im Behälter gesammelt. Dieser ist mit einem Deckel verschlossen, welcher mit einer gabelförmigen Haltevorrichtung eine Schnappverbindung eingeht und so den Behälter in seiner Position hält.

US 5 356 038 offenbart eine Befestigungsvorrichtung für einen Cremetubendispenser.

### Darstellung der Erfindung

Es ist deshalb eine Aufgabe der Erfindung, eine Vorrichtung zu schaffen, welche ein einfaches und sicheres Wechseln der Drainagebeutel erlaubt.

Diese Aufgabe löst ein Drainagebehälter mit den Merkmalen des Patentanspruchs 1.

Die erfindungsgemässe Befestigungsvorrichtung zur Befestigung des Aussenbehälters des Drainagebehälters weist ein Schnappschloss mit einem Schnappelement auf. Das Schnappelement ist vorzugsweise eine Blattfeder mit einer vorstehenden Nase.

Dank dem Schnappschloss wird der Aussenbehälter in seiner befestigten Position gehalten, auch wenn der Deckel mit dem Drainagebeutel entfernt wird. Der Drainagebeutel lässt sich somit einhändig schnell und sicher entfernen, ohne dass die ganze Einrichtung um ihn herum, insbesondere der Aussenbehälter, von Hand festgehalten werden muss.

Dank dem Schnappschloss lässt sich der Drainagebehälter nach wie vor auf einfache Weise befestigen. Die Befestigung ist vor allem erleichtert, wenn die Befestigungsvorrichtung eine Nut-/Nutsteinverbindung aufweist, so dass zum Befestigen des Drainagebehälters lediglich der Nutstein in die Nut eingeschoben werden muss, bis das Schnappschloss greift.

Vorzugsweise ist die Nut oder der Nutstein in einer Befestigungsklammer angeordnet. Diese Befestigungsklammer ist an einer Schiene befestigbar. Die Klammer erlaubt eine schnelle und sichere Verbindung mit einer Schiene.

Vorteilhaft ist, dass bekannte Befestigungsklammern weiter verwendet werden können und dass lediglich der Aussenbehälter des Drainagebehälters neu mit einem Schnappschloss versehen werden muss. Ist das Schnappschloss eine Blattfeder, ist der Wechsel von den bisherigen Produktelinien auf den erfindungsgemässen Drainagebehälter relativ einfach und deshalb kostengünstig.

Selbstverständlich kann jedoch auch die Befestigungsklammer mit dem Schnappelement versehen werden bzw. den Nutstein aufweisen.

In einer Ausführungsform ist der Drainagebehälter relativ kostengünstig herstellbar. Dieser Drainagebehälter weit einen Deckel auf, welcher ausschliesslich durch Auflage auf dem starren Aussenbehälter an diesem befestigt ist. Es hat sich gezeigt, dass die bisher verwendeten seitlichen Klammer zur Befestigung des Deckels nicht notwendig sind. Der im Aussenbehälter während der Drainage erzeugte Unterdruck genügt, um den Deckel und somit den Drainagebeutel sicher in seiner Position zu halten. Da somit weniger Teile hergestellt und montiert werden müsse, lässt sich der Drainagebehälter kostengünstiger herstellen.

Vorzugsweise wird der Deckel mit Griffen versehen, um den Deckel mit dem Drainagebeutel besser anheben zu können. Da sich im Falle eines Deckels aus Kunststoff die Griffe gemeinsam mit dem restlichen Deckel spritzen lassen, lassen sich die Herstellungskosten tief halten. Vorzugsweise sind zwei Griffe vorhanden, welche an zwei diametral gegenüberliegenden Seiten und seitlich am Deckel angeformt oder angebracht sind.

Vorzugsweise ist der Beutel mit dem Deckel fest verbunden, insbesondere verschweisst oder verklebt. Ein separates Kopfteil für den Beutel erübrigt sich somit. Auch dies senkt die Herstellungskosten.

In einer Ausführungsform ermöglicht der Drainagebehälter einen sicheren Betrieb. Dieser Drainagebeutel weist im Deckel ein Araschlusselecnent für die patientenseitige Drainageleitung auf, welches einen Winkel bildet. Der Winkel ist vorzugsweise ungefähr 45°. Es kann entweder der Anschlussstutzen selber gebogen sein oder es kann ein gebogenes Anschlussröhrchen in diesen eingesteckt sein.

Dank des Winkels ist die Gefahr, dass der patientenseitige Drainageschlauch geknickt wird und somit die Saugwirkung beeinträchtigt ist, vermindert. Es ist nicht mehr so kritisch, ob der Drainagebehälter auf einer bestimmten Höhe bezüglich des Patienten angeordnet ist. In einem vertretbaren Rahmen darf er sich nun auch durchaus höher oder tiefer als diese optimale Lage befinden.

Weitere vorteilhafte Ausführungsformen gehen aus den abhängigen Patentansprüchen hervor.

### Kurze Beschreibung der Zeichnung

Im folgenden wird der Erfindungsgegenstand anhand eines bevorzugten Ausführungsbeispiels, welches in der beiliegenden Zeichnung dargestellt ist, erläutert. Es zeigen:
- Figur 1: eine perspektivische Darstellung eines erfindungsgemässen Drainagebehälters von einer ersten Seite;
- Figur 2: eine perspektivische Darstellung des Behälters gemäss Figur 1 von einer zweiten Seite;
- Figur 3: eine perspektivische Darstellung des Behälters gemäss Figur 1 aus einer Richtung von oben;
- Figur 4: eine perspektivische Darstellung der erfindungsgemässen Befestigungsvorrichtung in einer im Vergleich zu Figur 1 vergrösserten Darstellung;
- Figur 5: die Befestigungsvorrichtung gemäss Figur 4 in einem gelösten Zustand und
- Figur 6: eine perspektivische Darstellung eines erfindungsgemässen Deckels mit Blick auf seine Unterseite.

### Wege zur Ausführung der Erfindung

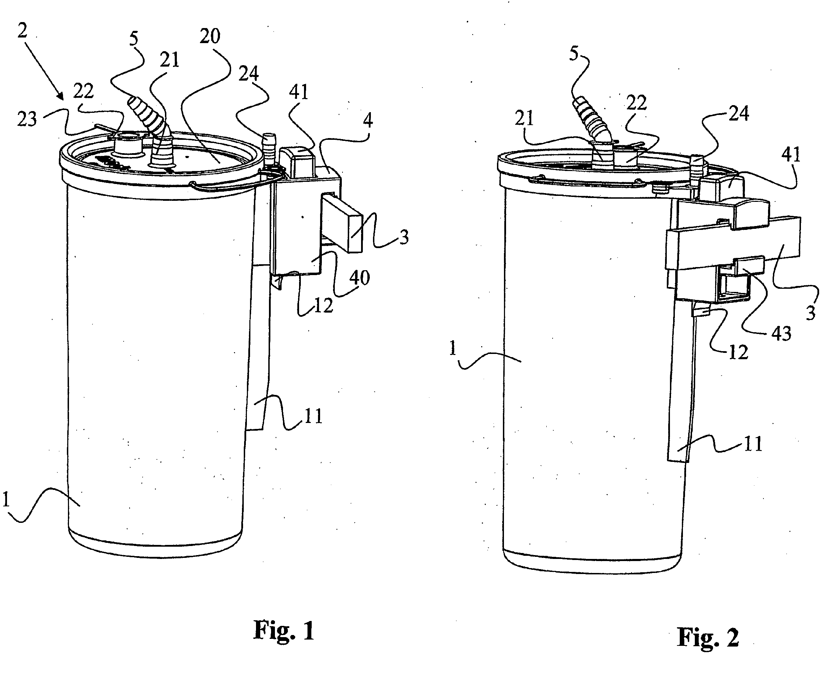

Die Figuren 1 bis 3 zeigen einen erfindungsgemässen Drainagebehälter aus zwei Seitenansichten und einer Ansicht aus einer oberen Richtung. Er weist einen starren, vorzugsweise durchsichtigen Aussenbehälter 1 und einen Verschlussdeckel 2 auf. Am Deckel 2 ist ein hier nicht sichtbarer Drainagebeutel befestigbar oder er ist vorzugsweise mit dem Deckel 2 fest verbunden, beispielsweise an dessen Umfangsrand angeschweisst oder verklebt. Der Drainagebeutel ist im Behälter 1 angeordnet und wird durch den auf dem Behälterrand aufliegenden Deckel 2 in seiner Position gehalten. Deckel 2, Beutel und Aussenbehälter 1 bestehen vorzugsweise aus Kunststoff, wobei der Deckel und der Aussenbehälter beispielsweise aus PC (Polycarbonat) oder PSU (Polysulfon) und der Beutel aus PE (Polyethylen) gefertigt sein können.

Im Deckel 2, bzw. in seiner kreisförmigen Grundfläche 20, sind mehrere Öffnungen vorhanden. Eine Öffnung ist von einem patientenseitigen Anschlussstutzen 21 umgeben, an welchen eine patientenseitige Drainageleitung anschliessbar ist. Dieser Anschlussstutzen 21 bildet eine Öffnung in den Drainagebeutel hinein. Ein Vakuumanschluss zur Verbindung mit der Vakuumquelle ist in den Figuren mit der Bezugsziffer 24 bezeichnet. In dargestellten Ausführungsbeispiel befindet sich dieser Anschluss in einem seitlich am Behälter 1 angeordneten Steg 11, wobei die Öffnung in den Behälterinnenraum führt. Es ist jedoch auch möglich, diesen Vakuumanschluss 24 im Deckel anzuordnen.

In die Öffnung des Anschlussstutzens 21 ist ein Anschlussröhrchen 5 eingesteckt, welches einen Winkel bildet. Vorzugsweise beträgt der Winkel annähernd 45°. An dieses Anschlussröhrchen 5 wird die patientenseitige Drainageleitung angeschlossen. Anschlussstutzen und -röhrchen lassen sich auch gemeinsam einstückig herstellen.

Es können noch weitere Öffnungen bzw. Stutzen vorhanden sein. Im hier dargestellten Beispiel ist im Deckel 2 ein Serienanschluss 22 angeordnet. Dieser dient dazu, zwei oder mehr Drainagebehälter in Serie zu schalten. Dabei werden die einzelnen Behälter mit Verbindungsleitungen miteinander verbunden, welche in diese Serienanschlüsse 22 gesteckt werden. Wird nur ein einzelner Drainagebehälter verwendet, so ist der Serienanschluss wie dargestellt mit einem Deckel 23 verschlossen, damit im Behälterinnenraum das notwendige Vakuum erzeugt werden kann. Vorzugsweise ist der Deckel 23 des Serienanschlusses 22 am restlichen Deckel 2 angeformt oder sonst wie mit ihm verbunden.

Am Deckel 2 sind Griffe 25 angeformt oder angebracht. Vorzugsweise sind Deckel und

Griffe gemeinsam einstückig ausgebildet. Diese Griffe 25 können nach oben abstehen. In der hier dargestellten bevorzugten Ausführungsform sind jedoch genau zwei Griffe 25 vorhanden, welche an diametral gegenüberliegenden Seiten des Deckels 2 an seinem Umfangsrand angeordnet sind. Dabei bilden sie mit dem restlichen Deckel eine gemeinsame Ebene.

Der Deckel 2 ist nicht speziell am Aussenbehälter 1 befestigt. Insbesondere sind keine Klammert für seine Befestigung vorhanden. Der Deckel 2 liegt lediglich auf dem oberen Rand des Aussenbehälters 1 auf und wird nur durch den bei Gebrauch im Behälter herrschenden Unterdruck in seiner Position gehalten.

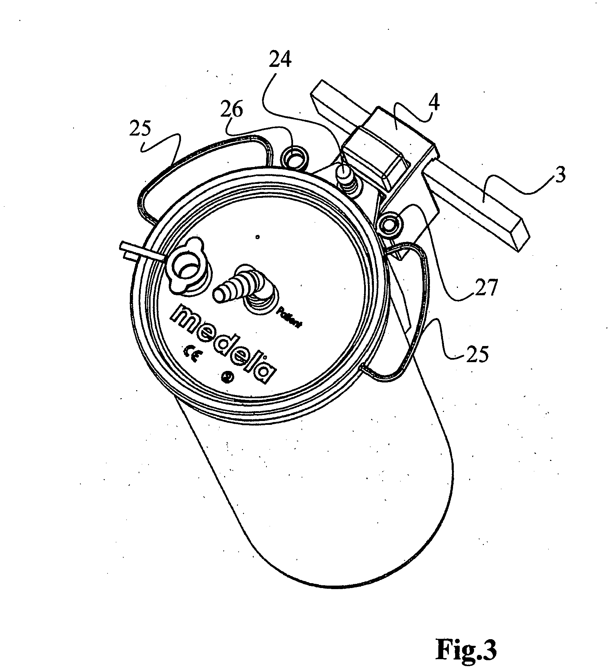

Wie in Figur 3 am besten erkennbar ist, sind am Deckel zwei Kappen 26, 27 angeformt. Diese Kappen 26, 27 lassen sich bei Gebrauch vom restlichen Deckel abbrechen und als Verschlüsse für den Anschlussstutzen 21 und den Vakuumanschluss 24 verwenden. Es ist jedoch auch möglich, keine oder nur eine Kappe anzubringen. Ferner kann eine Kappe so gestaltet sein, dass sie zum Verschliessen von verschiedenen Öffnungen dienen kann. Beispielsweise kann sie in einer Position den Anschlussstutzen 21 verschliessen und einer gedrehten Lage so geformt sein, dass sie den Vakuumanschluss 24 oder den Serienanschluss 22 verschliessen kann.

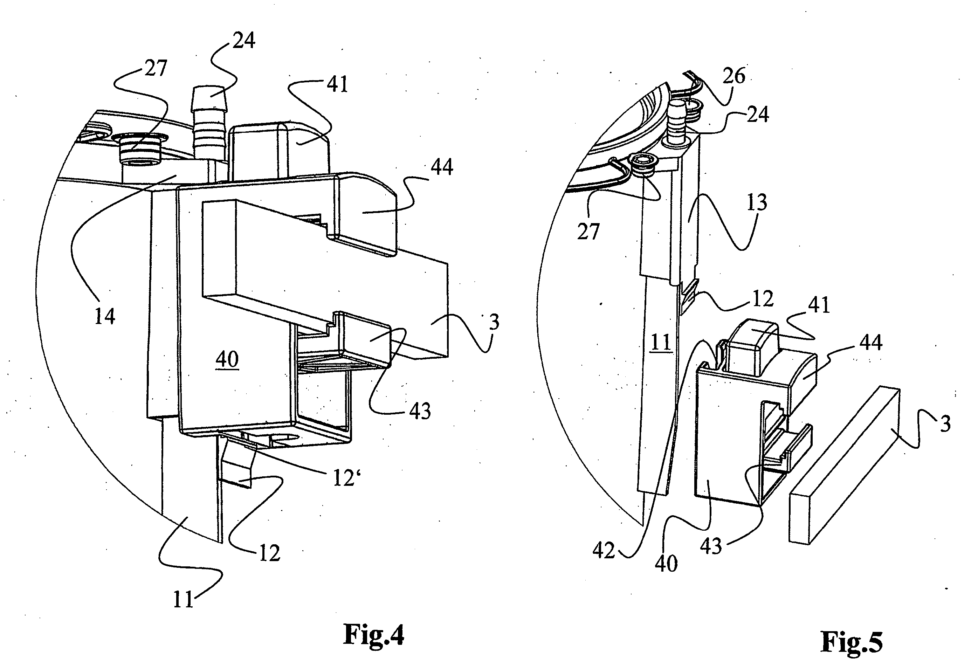

Der Aussenbehälter 1 weist vorzugsweise eine Kreiszylinderform auf. Am Mantel des Aussenbehälters 1 ist der erwähnte Steg 11 angeschweisst oder angeformt. Er steht nach aussen vor, und geht im oberen Bereich in einen Nutstein 13 über, wie dies in Figur 5 dargestellt ist. Der Nutstein 13 weist eine Längsrichtung auf, welche parallel zu einer Längsachse des Behälters 1 verläuft.

Das Gegenstück des Nutsteins 13 wird durch eine Nut 42 gebildet, welche in einem Befestigungselement bzw. einer Befestigungsklammer 4 verläuft. Dieses Befestigungselement 4 lässt sich an eine Schiene eines Krankenbetts, an eine Schiene einer Drainagevorrichtung, an eine Wandschiene oder an eine andere geeignete verlaufende Schiene befestigen. Die Schiene ist in den Figuren nur als kurzer Abschnitt gezeichnet.

Die erfindungsgemässe Befestigungsvorrichtung lässt sich nun in den Figuren 4 und 5 am besten erkennen. Das zugehörige Befestigungselement 4 weist einen Grundkörper 40 auf. Auf einer Seite des Grundkörpers 40 verläuft die erwähnte Nut 42 in senkrechter Richtung. Die andere Seite bildet die Klammer. Der obere Teil der Klammer ist hier fester Bestandteil des Grundkörpers 40 und wird durch eine nach unten gebogene feste Klaue 44 gebildet. Der untere Teil der Klammer ist eine nach oben gebogene untere, bewegbare Klaue 43, welcher federbelastet in vertikaler Richtung verschiebbar ist. Die Feder verläuft im Innern des Grundkörpers 40 und ist deshalb hier nicht sichtbar. Die bewegbare Klaue 43 ist mit einem Betätigungsknopfs, hier ein Druckknopf 41 verbunden, welcher die obere Seite des Grundkörpers 40 überragt und nach unten gedrückt werden kann. Er bewegt dadurch die Klaue 43 entgegen der Federkraft nach unten. Die Klaue 43 und die nach unten gebogene obere feste Klaue 44 bilden zusammen eine in waagrechter Richtung verlaufende Nut, in welche die Schiene 3 aufgenommen wird. Selbstverständlich kann auch die obere Klaue beweglich ausgebildet sein und die untere fest.

Die erfindungsgemässe Befestigungsvorrichtung umfasst ein Schnappschloss 12, welches hier durch eine Blattfeder 12 mit einer der Oberseite des Nutsteins 13 vorstehenden Nase 12' gebildet wird. Die Blattfeder 12 ist die nach unten gerichtete Verlängerung des Nutsteins 13, wobei sie schmaler ausgebildet ist als die grössere Breite des Nutsteins 13. Die Blattfeder 12 kann aus Metall oder Kunststoff oder einem anderen geeigneten Material gefertigt sein. Das Schnappschloss kann auch anders ausgestaltet sein als hier beschrieben. Wesentlich ist, dass es durch Zuschnappen fest schliesst, d.h. den Aussenbehälter an die Befestigungsklammer zwar lösbar aber fest fixiert.

Wie in Figur 5 erkennbar ist, wird die Befestigungsklammer 4 an der Schiene 3 befestigt und der Behälter 1 wird mit seinem Nutstein 13 von oben in die Nut 42 der Befestigungsklammer 4 eingeführt, bis er mit einer am oberen Ende des Nutsteins 13 angebrachten Platte 14 auf dem Grundkörper 40 aufliegt. In dieser Position ist das Schnappschloss zugeschnappt und die vorstehende Nase 12' der Blattfeder 12 hintergreift die Nut 42 des Befestigungselements 4. Damit der Behälter 1 wieder entfernt werden kann, muss die Feder 12 von Hand in Richtung Behälter 1 gedrückt werden, so dass der Nutstein 13 wieder nach oben aus der Nut 42 gezogen werden kann. Das Schnappschloss bildet somit eine Arretierung des Behälters und verhindert unerwünschte Zugbewegungen nach oben.

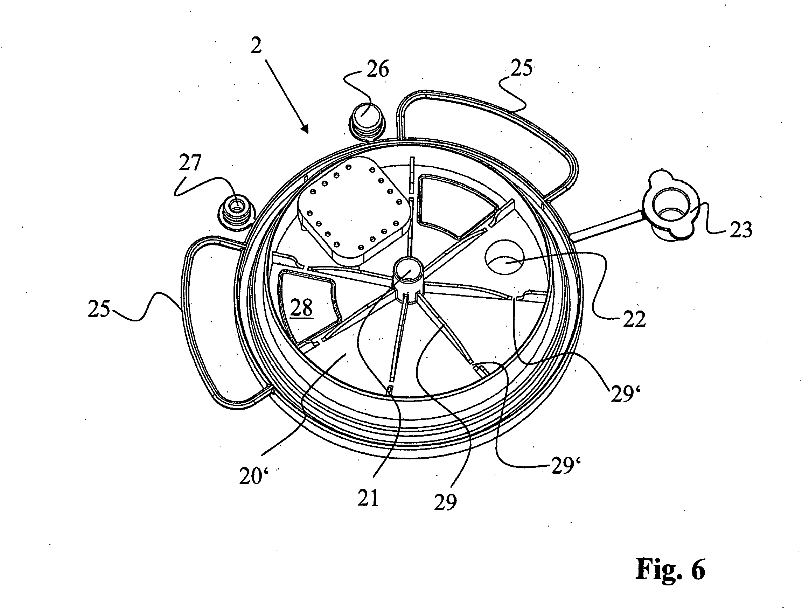

Im folgenden wird detaillierter auf den Deckel 2 eingegangen, wie er in Figur 6 dargestellt ist. Er weist auf seiner Unterseite, d.h. die zum Behälterinnenraum gerichteten Seite sternförmig angeordnete Rippen 29 auf, welche sich vorzugsweise bis annähernd an den Rand des Deckels erstrecken. Jede Rippe 29 ist jeweils mit mindestens einem Unterbruch 29' versehen, so dass die einzelnen Bereiche 20' der Deckelinnenfläche miteinander verbunden sind. Dadurch kann beim Reinigen des Deckels 2 keine Flüssigkeit liegen bleiben.

Um die Reinigung zu erleichtern, verfügt der Deckel 2 über mindestens einen, vorzugsweise mehrere herausbrechbare Bereiche 28, durch welche der Drainagebeutel schnell entleert werden kann. Hier sind es zwei derartige Bereiche 28. Damit der Beutel und der Deckel 2 zwecks sicherer Entsorgung in einer dafür geeigneten Waschmaschine gewaschen werden können, sind hier nicht dargestellte Positionierungshilfen vorhanden, damit der Deckel 2 stets in gleicher Lage und vor allem sicher in der Waschmaschine gehalten werden kann. Als Positionierhilfe dient vorzugsweise mindestens ein Loch, welches in einem Umfangsrand des Deckels 2 angeordnet ist.

Die erfindungsgemässe Befestigungsvorrichtung ermöglicht ein einfaches und sicheres Wechseln des Drainagebeutels. Der zugehörige Drainagebehälter lässt sich kostengünstig herstellen und gewährt einen sicheren Betrieb.

### Bezugszeichenliste

- 1: Aussenbehälter
- 11: Steg
- 12: Blattfeder
- 12': vorstehende Nase
- 13: Nutstein
- 14: Platte

- 2: Verschlussdeckel
- 20: Grundfläche
- 20': Bereich
- 21: patientenseitiger Anschlussstutzen
- 22: Serienanschluss
- 23: Deckel des Serienanschlusses
- 24: Vakuumanschluss
- 25: Griff
- 26: erste Kappe
- 27: zweite Kappe
- 28: herausbrechbarer Bereich
- 29: Rippe
- 29': Unterbrechung

- 3: Schiene

- 4: Befestigungsklammer
- 40: Grundkörper
- 41: Druckknopf
- 42: Nut
- 43: bewegbare Klaue
- 44: feste Klaue
- 5: Anschlussröhrchen

## Patentansprüche

1. Drainagebehälter zum Auffangen von abgesaugten Körperflüssigkeiten mit einer Befestigungsvorrichtung (4, 13), wobei der Drainagebehälter einen starren Aussenbehälter (1), einen diesen verschliessenden Deckel (2) und einen am Deckel (2) anbringbaren bzw. angebrachten Drainagebeutel zur Aufnahme der Körperflüssigkeiten aufweist und wobei die Befestigungsvorrichtung eine Einrichtung umfasst, welche eine Arretierung des Drainagebehälters (1) bildet, **dadurch gekennzeichnet, dass** die Befestigungsvorrichtung (4, 13) zur Befestigung des Aussenbehälters (1) an einer Schiene dient, so dass der Drainagebehälter an dieser Schiene angehängt werden kann, und dass die Einrichtung ein Schnappschloss (12) ist, welches bei Entfernung des Deckels (2) mit dem Drainagebeutel den Aussenbehälter (1) in seiner befestigten Position hält.

2. Drainagebehälter nach Anspruch 1, wobei das Schnappschloss (12) eine Nut-/Nutsteinverbindung (42, 13) aufweist und wobei an einem unteren Ende der Verbindung eine Blattfeder (12) mit einer vorstehenden Nase (12') angeordnet ist, welche eine untere Kante der Nut (42) oder des Nutsteins (13) hintergreift,

3. Drainagebehälter nach einem der Ansprüche 1 oder 2, wobei der Nutstein (13) am Aussenbehälter (1) und die Nut (42) an einem Befestigungselement (4) oder umgekehrt angeordnet ist.

4. Drainagebehälter nach Anspruch 3, wobei das Befestigungselement eine Befestigungsklammer (4) ist.

5. Drainagebehälter nach Anspruch 4, wobei die Befestigungsklammer (4) einen Grundkörper (40) aufweist, welcher auf einer Seite die Nut (42) oder den Nutstein (13) und auf einer gegenüberliegenden Seite eine federbelastete bewegbare Klaue (43) aufweist.

6. Drainagebehälter nach Anspruch 5. wobei die bewegbare Klaue (43) mittels eines Druckknopfs (41) bewegbar ist.

7. Drainagebehälter nach Anspruch 6, wobei die bewegbare Klaue (43) in einer Richtung parallel zu einer Längsrichtung der Nut (42) bzw. des Nutsteins (13) bewegbar ist.

8. Drainagebehälter nach einem der Ansprüche 2 bis 7, wobei die Blattfeder (12) am Aussenbehälter (1) angeordnet ist.

## Claims

1. A drainage container for collecting body fluids extracted by suction with a fastening device (4, 13), wherein the drainage container has a rigid outer container (1), a lid (2) closing the outer container (1) and a drainage bag for receiving the body fluids, and wherein the drainage bag can be attached or is attached to the lid (2), and wherein the fastening device comprises a system, which is an arrest of the drainage container (1), **characterized in that** the fastening device (4, 13) serves to fasten the outer container (1) to a rail, so that the drainage container is suspended from this rail and that the system is a snap lock (12), which holds the outer container (1) in its fastened position when the lid (2) with the drainage bag is removed.

2. The drainage container as claimed in claim 1, wherein the snap lock (12) comprises a slot/slot pin connection (42, 13) and wherein a leaf spring (12) having a projecting nose (12') is arranged at a lower end of the connection and engages behind a lower edge of the slot (42) or of the slot pin (13).

3. The drainage container as claimed in one of claims 1 or 2, wherein the slot pin (13) is arranged on the outer container (1) and the slot (42) is arranged on a fastening element (4) or vice versa.

4. The drainage container as claimed in claim 3, wherein the fastening element is a fastening clamp (4).

5. The drainage container as claimed in claim 4, wherein the fastening clamp (4) has a basic body (40) having on one side the slot (42) or the slot pin (13) and on an opposing side a spring-loaded movable claw (43).

6. The drainage container as claimed in claim 5, wherein the movable claw (43) can be moved by means of a push-button (41).

7. The drainage container as claimed in claim 6, wherein the movable claw (43) is movable in a direction parallel to a longitudinal direction of the slot (42) or the slot pin (13).

8. The drainage container as claimed in one of claims 2 to 7, wherein the leaf spring (12) is arranged on the outer container (1).

## Revendications

1. Récipient de drainage destiné à recueillir des fluides corporels aspirés, doté d'un dispositif de fixation (4, 13), le récipient de drainage comprenant un récipient extérieur rigide (1), un couvercle (2) fermant ce dernier et un sachet de drainage destiné à recevoir les fluides corporels, qui peut être attaché ou qui est attaché au couvercle (2), et le dispositif de fixation comportant un système qui forme un blocage du récipient de drainage (1), **caractérisé en ce que** le dispositif de fixation (4, 13) sert à la fixation du récipient extérieur (1) à un rail, de telle sorte que le récipient de drainage puisse être accroché à ce rail, et **en ce que** le système est un verrou à encliquetage (12) qui maintient le récipient extérieur (1) dans sa position fixée lorsque le couvercle (2) est retiré conjointement avec le sachet de drainage.

2. Récipient de drainage selon la revendication 1, dans lequel le verrou à encliquetage (12) comprend une liaison rainure/coulisseau (42, 13), et dans lequel un ressort à lame (12) doté d'un ergot saillant (12') est disposé sur une extrémité inférieure de la liaison, lequel ergot vient en prise par l'arrière avec un bord inférieur de la rainure (42) ou du coulisseau (13).

3. Récipient de drainage selon la revendication 1 ou 2, dans lequel le coulisseau (13) est disposé sur le récipient extérieur (1) et la rainure (42) est disposée sur un élément de fixation (4) ou vice versa.

4. Récipient de drainage selon la revendication 3, dans lequel l'élément de fixation est une agrafe de fixation (4).

5. Récipient de drainage selon la revendication 4, dans lequel l'agrafe de fixation (4) comprend un corps de base (40) qui comprend d'un côté la rainure (42) ou le coulisseau (13) et du côté opposé une patte (43) mobile sollicitée par un ressort.

6. Récipient de drainage selon la revendication 5, dans lequel la patte (43) mobile peut être déplacée au moyen d'un bouton-poussoir (41).

7. Récipient de drainage selon la revendication 6, dans lequel la patte (43) mobile peut être déplacée dans une direction parallèle à une direction longitudinale de la rainure (42) ou du coulisseau (13).

8. Récipient de drainage selon l'une quelconque des revendications 2 à 7, dans lequel le ressort à lame (12) est disposé sur le récipient extérieur (1) .
